# EUROPEAN PATENT SPECIFICATION

(11) **EP 0 347 461 B1**
(45) Date of publication and mention of the grant of the patent: **11.08.1993**
(21) Application number: 88902543.3
(22) Date of filing: 11.03.1988
(51) Int. Cl.: A61K 9/52, A61K 9/54

(54) **A SUSTAINED-RELEASE SOLID MEDICAMENT FORM AND A METHOD FOR THE PREPARATION THEREFOF**
FESTE ARZNEIFORM MIT VERZÖGERTER FREISETZUNG UND METHODE FÜR DEREN HERSTELLUNG
MEDICAMENT SOUS FORME SOLIDE A LIBERATION ENTRETENUE ET PROCEDE DE PREPARATION

(30) Priority: 23.12.1987 JP 3265/88
(43) Date of publication of application: 27.12.1989
(73) Proprietor: SHIN-ETSU CHEMICAL CO., LTD., Chiyoda-ku Tokyo 100 (JP)
(72) Inventor: SEKIGAWA, Fujio, Omiya-shi Saitama 330 (JP); KOKUBO, Hiroyasu, Niigata 942 (JP); ONDA, Yoshiro, Tokyo 203 (JP)
(74) Representative: Virr, Dennis Austin
(86) International application number: JP8800258
(87) International publication number: WO8905634

(56) References cited:
- EP-A- 0 013 262
- EP-A- 0 013 566
- EP-A- 0 164 959
- US-A- 2 540 979

## Description

### Field of Technology

The present invention relates to a method for the preparation of a sustained-release granular solid medicament form of which the concentration of the effective pharmaceutical ingredient in blood can be maintained at an effective level to exhibit the desired medical effect over a long time by means of a single administration.

### Background Technology

Solid medicament forms for sustained release in the prior art include granules, capsules filled with granules, tablets and the like, of which the largest number of the hitherto commercialized medicament products are in the granular form by virtue of the advantage of granules that any desired dose of the medicament can be administered to a patient without dividing, as is sometimes necessary in the administration of tablets. As one method for the preparation of a typical sustained-release granular solid medicament form, the surface of granules shaped from a mixture composed of an effective ingredient, excipient and binder is coated with a solution of a wax-like coating material in an organic solvent, followed by drying. The factors which provide proper selection of the position in the digestive tract where the effective ingredient is released and control of the sustainability of the concentration of the ingredient in blood for a desired length of time include the proportion of the components in the mixture forming the granules, the solubility behaviour of the binder in the digestive fluids, the amount of the wax-like coating material and so on. Another type of a sustained-release granular medicament form is known which is mainly composed of an effective ingredient and a wax or polymeric substance insoluble in water but soluble in organic solvents. It is of course indispensable in the process of granulation thereof to use an organic solvent for dissolving the wax or polymeric substance.

The sustained-release solid medicament forms in the prior art have various problems in respect of the performance as a medicine and in the method for the preparation thereof. For example, the use of an organic solvent involves problems including danger of fire and explosion, toxicity against the workers in the production of the medicament, eventual residual amount of the solvent in the finished medicament form and so on. The residual amount of the solvent in the finished medicament form can be decreased only by a substantial extension of the drying time in the coating process, leading to a disadvantageous decrease in productivity due to the increase of the overall time taken for the production; there is also the disadvantage of possible decomposition of the effective ingredient by prolonged heating.

Alternatively, a method of water-based film coating has been developed in which the coating solution is prepared from a water-soluble polymeric material without using any organic solvent and the method is widely utilized in the coating of ordinary solid medicament forms of tablets and granules. No practical procedure has yet been proposed, however, of the water-based film coating method in the preparation of sustained-release solid medicament forms. The reason for this is that the film coating method has been developed mainly with the objects of preventing decrease in the activity of the effective ingredient, and reducing the bitterness and improving the appearance of the product, while the object of a sustained-release solid medicament form to ensure a sufficiently high concentration of the effective ingredient in blood over a length of time has been achieved solely by means of a wax-like material insoluble or hardly soluble in water.

On the other hand, Japanese Patent Kokai No. 58-110513 teaches a composition for sustained-release solid medicament forms of which the carrier base is a hydroxypropyl methyl cellulose or a mixture thereof with 30% by weight or less of an ethyl cellulose and/or sodium salt of carboxymethyl cellulose. The medicament form disclosed there is prepared by compression molding, so that the object thereof is basically a solid medicament form in the form of tablets. Although such a solid medicament form is suitable for the purpose of sustainedly releasing the effective ingredient, a disadvantage thereof is that, when the tablets are divided into fragments with the object of adjusting the dose to a patient, the performance of the medicament relative to the sustained releasability of the ingredient is totally altered depending on the divided condition of the tablets. In addition, the composition disclosed there is not suitable as a material for granules because sufficiently dense granules can hardly be prepared therefrom, so that the effective ingredient is released prematurely and does not meet the object of a sustained-release solid medicament form.

In connection with the method of water-based film coating of granules and tablets to impart sustained releasability thereto by use of a cellulose ether as the base material of the coating film, no satisfactory sustained releasability has ever been obtained due to the problems in the properties of the base material and the conditions of the working procedure suitable for film coating. When carrier granules are coated in a conventional manner, in particular, adherence of the granules proceeds in the course of coating to cause so-called agglomeration unless the concentration of the cellulose ether in the coating solution is extremely low, so that film coating of granules with a cellulose ether cannot be practised industrially by solving the above-mentioned problem. In this regard, the inventors have previously proposed a method of film coating by using a hot-water dispersion of a high-molecular cellulose ether which, in the prior art, can hardly be used for film coating.

EP-A-0164959 discloses a sustained-release composition in the form of beads within a capsule of gelatin or the like. Each bead comprises an inert core with a coating of medicament particles which in turn is covered by a further coating of three different polymers with different solubility profiles.

EP-A-0013566 discloses a method of providing an enteric coating on solid dosage forms which entails forming an aqueous coating liquid by dispersing a powder of a hydroxypropylmethylcellulose phthalate in an aqueous medium containing triacetin.

EP-A-0210540 describes what it calls "time-controlled explosion systems" wherein the drug release from a coated bead is caused by explosion of a membrane after a predetermined time.

WO-A-88/00046 discloses delayed release of an active ingredient which has been distributed in or on pellets, by coating the pellets with a permeable membrane.

### Disclosure of the Invention

In the course of the investigations leading to the above-mentioned method of film coating by using a hot water dispersion of a high-molecular cellulose ether, it has been unexpectedly discovered that a quite satisfactory sustained-release granular solid medicament form can be obtained when carrier granules are coated with the above-mentioned coating dispersion which also contains the effective pharmaceutical ingredient dissolved or dispersed therein so as to provide the carrier granules with a dense coating layer composed of the hot water-insoluble cellulose ether and the effective ingredient from which the effective ingredient is released at an efficiently controlled rate.

Thus the product which is prepared by the method according to the present invention is a sustained-release granular solid medicament form of which each granule is composed of:
(a) a core granule formed from a carrier comprising at least a major proportion of an excipient; and
(b) a coating layer on the surface of the granules which coating layer is formed of a cellulose ether which is insoluble in water above a predetermined temperature and which coating layer further contains a pharmaceutically effective ingredient.

The method according to the present invention comprises the steps of:
(A) granulating a carrier composed of at least a major proportion of said excipient to form said core granules;
(B) preparing a coating liquid containing from 5 to 30 per cent by weight of the cellulose ether dispersed therein, by dispersing said cellulose ether and by dispersing or dissolving said pharmaceutically effective ingredient, both in water at a temperature above said predetermined temperature;
(C) coating the core granules with the coating liquid to form coated granules; and
(D) drying the coated granules.

### Best Mode Embodiments to Practise the Invention

The mechanism by which the above-described granular solid medicament form exhibits sustained releasability of the effective ingredients is not quite clear. It is presumed that, when the coating layer formed mainly of a cellulose ether and containing the effective ingredients is brought into contact with the digestive fluid, the cellulose ether absorbs the fluid and is converted into a layer of a hydrogel which is gradually dissolved in the digestive fluid to release the effective ingredient or through which the effective ingredient is gradually leached out into the digestive fluid. Accordingly, the sustained releasability of the effective ingredient from the granules can be controlled by appropriately selecting the hot-water-insoluble cellulose ether forming the layer of hydrogel depending on the nature of the effective ingredient and, optionally, by providing an overcoating layer with a wax-like coating material according to need. The core granules made mainly of an excipient serve to control the final disintegration time of the medicament form in the digestive tract.

Several kinds of cellulose ethers are suitable as the hot-water-insoluble cellulose ether used in the invention, including hydroxypropyl methyl cellulose, methyl cellulose, hydroxypropyl cellulose, hydroxyethyl methyl cellulose and the like. One of the important characteristics of these cellulose ethers is that they are insoluble in hot water but soluble in cold water so that, when an aqueous dispersion of the powdery cellulose ether at a sufficiently high temperature is gradually cooled, dissolution of the cellulose ether particles takes place at a predetermined temperature and the dispersion is converted into an aqueous solution showing a rapid increase of viscosity. The above-mentioned critical temperature below which the cellulose ether is dissolved in water is called the "solubilization temperature", which is a value inherent in each type of cellulose ether products. For example, methyl cellulose and hydroxypropyl cellulose have a solubilization temperature of 35 to 45°C while different grades of hydroxypropyl methyl cellulose have different solubilization temperatures of 45 to 55°C for type 2910, 50 to 60°C for type 2906 and 60 to 70°C for type 2208 of hydroxypropyl methyl cellulose according to the classification in Japanese Pharmacopoeia.

The aqueous dispersion of a hot-water-insoluble cellulose ether is prepared by adding the powdery cellulose ether to water at a temperature higher than the above-mentioned solubilization temperature of the respective cellulose ether under agitation and the aqueous dispersion of a cellulose ether once prepared should be kept at a temperature not lower than the solubilization temperature of the cellulose ether throughout until the dispersion is used as a coating liquid for the core granules. On the contrary, cellulose ether powders should never be added to cold water to be dispersed therein because the particles of the cellulose ether dispersed in cold water become at least partly dissolved in water in the course of temperature increase of the dispersion so that the dispersion acquires a gel-like consistency and can no longer be used as a coating liquid for the core granules. The aqueous dispersion of a cellulose ether used as the coating liquid should contain from 5 to 30% by weight of the powdery cellulose ether. When the content thereof in the aqueous dispersion is too high, the dispersion may have a slurry-like consistency, eventually causing clogging of the spray gun nozzles used in the coating work. When the content is too low on the other hand, coating layers of a desired thickness can be formed only by continuing the coating work for an unduly long time, which is economically disadvantageous.

The hot-water-insoluble cellulose ether used in the inventive method should preferably have such a particle size distribution that an at least 90% by weight portion thereof passes through a No. 100 screen having a mesh opening of 149 µm as specified in the Japanese Pharmacopoeia. When the powder contains coarser particles in an excessively large amount, no satisfactory coating films can be formed on the core granules due to incomplete coalescence of the particles in the coating layer.

The pharmaceutically effective ingredient dissolved or dispersed in the aqueous dispersion of the cellulose ether is not particularly limitative and any medicinal compound can be used when it should desirably be released sustainedly in the digestive tract for medical purposes. Examples of medicinal compound usable in the invention include theophylline, aspirin, nifedipine, chlorpheniramine maleate, propantheline bromide, trihexyphenydyl hydrochloride, diclofenac sodium, indomethacin, nitroglycerin, potassium chloride, iron (II) sulfate, iron (II) fumarate and the like. The amount of the pharmaceutically effective ingredient dissolved or dispersed in the coating liquid should of course be adequately selected depending on the desired rate of sustained release of the medicine, total dose and other factors but it is usually in the range from 0.1 to 50% by weight based on the amount of the cellulose ether dispersed therein. When the pharmaceutically effective ingredient is insoluble in neutral water, it is of course dispersed in water to give a coating liquid, if necessary after pulverization in order to increase the absorptivity of the medicine in the digestive organ. The pharmaceutically effective ingredient can be added to the coating liquid in any convenient procedure without particular limitation. For example, it can be added as such to a hot aqueous dispersion of the cellulose ether prepared beforehand or, alternatively, it can be dissolved or dispersed in hot water and the hot solution or dispersion is then admixed with the aqueous dispersion of the cellulose ether.

The coating liquid, which is an aqueous dispersion of a cellulose ether containing a pharmaceutically effective ingredient either dissolved or dispersed therein, may optionally contain various kinds of additives according to need, including water-soluble polymers and surface active agents to improve the dispersibility of the cellulose ether particles and a water-insoluble effective ingredient, colouring agents such as edible dyes and edible lake pigments, extender pigments such as talc and a finely divided silica powder, plasticizers such as polyethylene glycol and triethyl citrate, flavours such as vanilla extract and menthol, and others. The sustained releasability of the pharmaceutically effective ingredient can be controlled by admixing the coating liquid with saccharides, e.g. lactose, and waxes, e.g. beeswax and carnauba wax.

The core granules as the carrier of the above-described coating layer are shaped from an excipient such as lactose, starch, microcrystalline cellulose and the like or a mixture mainly composed of such an excipient with optional addition of a part of the pharmaceutically effective ingredient, colouring agents, taste and flavour improvers and the like. These materials are kneaded together with addition of a small volume of water and granulated by extrusion into a columnar form by use of an extrusion granulator followed by drying in a fluidized drier, although the method of granulation is not particularly limitative. The core granules preferably have such a particle size distribution that at least 85% by weight of the particles can pass through a No. 42 screen having a mesh opening of about 355 µm specified in Japanese Pharmacopoeia. When the particle size distribution of the core granules is coarser than above, some inconveniences are caused in handling of the medicament form and the medicament form is less acceptable to the patient. When the granules are too fine on the other hand, the resultant solid medicament form may be unsatisfactory in respect of the sustained releasability of the pharmaceutically effective ingredient.

The core granules are then coated with the coating liquid, which is a hot aqueous dispersion of the cellulose ether containing the pharmaceutically effective ingredient dissolved or dispersed therein. The coating procedure may be conventional by use of any known coating machine in the prior art, such as fluidized coating machines, pan coaters having a mechanism of aeration for drying and the like used in the process of film coating of granules in the prior art. The coating liquid must be kept at a sufficiently high temperature to prevent premature dissolution of the cellulose ether so that the coating apparatus should preferably be provided with an appropriate means for heating or heat insulation of the coating liquid. The coating amount of the coating layer containing the effective ingredient is preferably in the range from 5 to 20% by weight based on the core granules.

The granular medicament obtained in the above-described manner is quite satisfactory in most cases in its performance as a sustained-release solid medicament form. It may, however, optionally be further provided with a thin overcoating layer of a wax-like material when an increased possibility of controlling the sustained releasability is desired. Thus the granules after the above-described coating process may be admixed with a small amount of a wax-like material and subjected to a heat treatment at a temperature higher than the melting point of the wax-like material. In this regard, the wax-like material used as the material for overcoating is preferably selected from those having a melting point in the range from 40 to 90°C or, preferably, from 55 to 70°C including waxes, e.g. paraffins, beeswax and carnauba wax, higher alcohols, higher fatty acids and esters thereof, glycerin fatty acid esters, polyethylene glycols and the like. When the melting point of the wax-like material is too low, the overcoated granules may eventually adhere to each other when they are stored under ordinary conditions. When the melting point of the wax-like coating material is too high on the other hand, the process of coating must be performed by heating the blend of the granules and the wax-like material at an unduly high temperature, with the risk of eventual denaturation of the pharmaceutically effective ingredient contained in the coating layer of the cellulose ether depending on the thermal stability of the ingredient. The heat treatment is performed by fluidizing the blend of the granules and the wax-like coating material with hot air in a fluidized coating machine or by blowing hot air at the blend under rotation in a coating pan of a pan coater. Although the wax-like coating material may be introduced into the coating machine in any desired form such as fine powder, granules, blocks and the like, a granular form is preferred in respect of the convenience in handling. The temperature of the hot air for the fluidization of or blown at the blend is usually at or in the vicinity of the melting point of the wax-like coating material though not limited thereto. By this wax treatment, a thin overcoating layer of the wax-like coating material is formed on the surface of the granules, to exhibit a retarding effect on the releasing rate of the pharmaceutically effective ingredient contained in the underlying layer. Accordingly, the retarding effect on the releasing rate of the pharmaceutically effective ingredient from the inventive granular medicament form is increased by the increase in the amount of overcoating with the wax-like coating material. Usually, the amount of overcoating with the wax-like coating material is preferably in the range from about 0.2% to about 30% by weight based on the granular solid medicament form of the invention. Regardless of the above-described overcoating with a wax-like coating material, the inventive granular sustained-release solid medicament form may optionally be also provided with another type of an overcoating layer such as gastrosoluble and enterosoluble coating films depending on the particular requirement for the medicament.

In the following, the sustained-release granular solid medicament form of the invention and the method for the preparation thereof are described in more detail by way of examples, in which the terms of "%" and "parts" all refer to "% by weight" and "parts by weight" respectively.

### Example 1.

A coating liquid was prepared by adding and dispersing 15 parts of a hydroxypropyl methyl cellulose in 82 parts of hot water at 80°C with agitation and then 3 parts of indomethacin with further continued agitation. The hydroxypropyl methyl cellulose was a commercial product (60SH4000, manufactured by Shin-Etsu Chemical Co.), of which a 2% aqueous solution had a viscosity of 4.25 Pa.s (4250 centipoise) at 20°C, having such a particle size distribution that 99% of the particles could pass a No. 100 screen and a solubilization temperature of 48°C according to the Japanese Pharmacopoeia.

Separately, a mixture of 97 parts of lactose and 3 parts of a hydroxypropyl cellulose (HPC-EFP, manufactured by Shin-Etsu Chemical Co.) was kneaded with addition of a small volume of water and granulated into columnar granules passing through a screen of 0.8 mm diameter mesh openings by use of an extrusion granulator followed by drying for 2 hours in a fluidized drying machine with the fluidizing air at 80°C and sorting of the lengths by passing the granules through a granule sorter.

The granules prepared above were subjected to coating with the coating liquid. Thus, 3 kg of the granules were charged into a fluidized coater (Model Glatt WSG-5, manufactured by Ohgawara Seisakusho Co.) and coated with the coating liquid for 200 minutes under the conditions including: the temperature of the fluidizing air of 80°C; temperature of the exhaust of 47 to 50°C; and feed rate of the coating liquid of 50 g/minute followed by drying for 30 minutes with the fluidizing air at 80°C. In this manner, a sustained-release granular solid medicament form was obtained which contained 61 mg of of indomethacin per g.

For comparison, rapid-release granules of indomethacin for control test were prepared in the following manner. Granules were prepared from a blend composed of 61 parts of indomethacin, 630 parts of lactose, 279 parts of corn starch and 30 parts of the same hydroxypropyl cellulose as used above in essentially the same procedure as in the above-described granulation procedure of the core granules of lactose and the hydroxypropyl cellulose. The granules also contained 61 mg of indomethacin per g.

Gelatin-made capsules were filled each with 410 mg of the above-prepared inventive granules or comparative granules and subjected to a releasing test of the indomethacin according to the procedure for the releasing test in indomethacin capsules specified in Japanese Pharmacopoeia, to determine the % release of the ingredient as a function of time. The results are shown in Table 1 below, from which it is clear that the granules of the invention are quite satisfactory as a sustained-release solid medicament form.

**Table 1**

| Testing time, minutes | Released indomethacin, % | |
|---|---|---|
| | Sustained-release granules (inventive) | Rapid-release granules (control) |
| 30 | 28 | 87 |
| 60 | 48 | 100 |
| 120 | 63 | - |
| 180 | 81 | - |
| 240 | 94 | - |

### Example 2.

A coating liquid was prepared by first dispersing 10 parts of a hydroxypropyl methyl cellulose in 89 parts of hot water at 80°C with agitation and then adding and dissolving 1 part of chlorpheniramine maleate with further continued agitation. The hydroxypropyl methyl cellulose was a commercial product (90SH8000, manufactured by Shin-Etsu Chemical Co.), of which a 2% aqueous solution had a viscosity of 8.86 Pa.s (8860 centipoise) at 20°C, having such a particle size distribution that 98% of the particles could pass a No. 100 screen specified in Japanese Pharmacopoeia and a solubilization temperature of 64°C.

This coating liquid was used for coating of the same core granules as prepared in Example l. Thus, 3 kg of the core granules were introduced into the same fluidized coater as used in Example l and coated with the coating liquid for 200 minutes under the conditions including: the temperature of the fluidizing air of 80°C; temperature of the exhaust of 46 to 48°C; and feed rate of the coating liquid of 50 g/minute followed by drying for 30 minutes with the fluidizing air at 80°C and then wax treatment with fluidization under the same conditions after introducing 400 g of a bleached beeswax of Japanese Pharmacopoeia having a melting point of 60 to 67°C as a wax-like coating material in several portions. The thus-obtained sustained-release granular solid medicament form contained 22 mg of chlorpheniramine maleate per g.

For comparison, rapid-release granules of chlorpheniramine maleate for control test were prepared from a blend of 22 parts of chlorpheniramine maleate, 848 parts of lactose, 100 parts of a low-substitution hydroxypropyl cellulose (LH-21, manufactured by Shin-Etsu Chemical Co.) and 30 parts of the same hydroxypropyl cellulose as used in Example 1 in essentially the same procedure as in the granulation of the core granules in Example 1. The comparative granules contained 21 mg of chlorpheniramine maleate per g.

Gelatin-made capsules were filled each with 364mg of the above-prepared inventive granules or 381 mg of the comparative granules and subjected to a releasing test of the chlorpheniramine maleate by the first method of releasing test specified in Japanese Pharmacopoeia using the first fluid as the test solution. The amount of the released ingredient was determined by spectrophotometric measurement of the test solution at a wavelength of 262 nm, to give the results of the released amount of the chlorpheniramine maleate in % shown in Table 2 below, from which it is clear that the granules of the invention are quite satisfactory as a sustained-release solid medicament form.

**Table 2**

| Testing time, hours | Released chlorpheniramine maleate, % | |
|---|---|---|
| | Sustained-release granules (inventive) | Rapid-release granules (control) |
| 1 | 8 | 100 |
| 2 | 19 | - |
| 3 | 32 | - |
| 4 | 45 | - |
| 5 | 71 | - |
| 6 | 93 | - |

### Possibility of Industrial Utilization

To summarize the advantages obtained by the present invention, the problems in the prior art sustained-release solid medicament forms accompanying the use of organic solvents can be completely solved by the invention. In addition, the invention provides the following advantages.
(1) The granular sustained-release solid medicament form can be imparted with well-controlled releasability of the pharmaceutically effective ingredient, to meet the exact medical requirements.
(2) The coating procedure can be performed almost without the problem of agglomeration of the granules and granules of any desired coating amount can be easily prepared within a short working time.
(3) The activity of the pharmaceutically effective ingredient can be maintained sustainedly with good controllability because a cellulose ether of any desired molecular weight can be used without particular problems in the coating operation.

## Claims

1. A method for the preparation of a sustained-release granular solid medicament form of which each granule is composed of:
(a) a core granule formed from a carrier comprising at least a major proportion of an excipient; and
(b) a coating layer on the surface of the granule, which coating layer is formed of a cellulose ether which is insoluble in water above a predetermined temperature and which coating layer further contains a pharmaceutically effective ingredient,
which method comprises the steps of:
(A) granulating a carrier composed of at least a major proportion of said excipient to form said core granules;
(B) preparing a coating liquid containing from 5 to 30 per cent by weight of the cellulose ether dispersed therein, by dispersing said cellulose ether and by dispersing or dissolving said pharmaceutically effective ingredient, both in water at a temperature above said predetermined temperature;
(C) coating the core granules with the coating liquid to form coated granules; and
(D) drying the coated granules.

2. A method as claimed in Claim 1, wherein the powder of the cellulose ether has such a particle size distribution that at least 90 per cent by weight of the particles pass through a screen having mesh openings of 149 µm.

3. A method as claimed in Claim 1, wherein the coating liquid contains from 0.1 per cent to 50 per cent by weight of the pharmaceutically effective ingredient based on the weight of the cellulose ether.

## Patentansprüche

1. Verfahren zur Herstellung einer verzögernd freisetzenden granularen festen Arzneimittelform, wobei jedes Korn bzw. Granulat aus folgenden Komponenten besteht:
(a) einem aus einem Träger gebildeten Kornkern, der einen zumindest überwiegenden Anteil eines Placebos enthält; und
(b) einer Beschichtungslage auf der Oberfläche des Korns, welche aus einem Zelluloseether besteht, der oberhalb einer vorbestimmten Temperatur wasserunlöslich ist und welche weiterhin einen pharmazeutisch wirksamen Bestandteil enthält;
welches Verfahren folgende Schritte umfaßt:
(A) Granulieren eines Trägers, der zumindest aus einem überwiegenden Anteil aus dem Placebo besteht, zum Bilden der Kornkerne;
(B) Präparation einer Beschichtungsflüssigkeit, die 5 bis 30 Gew.-% des darin dispergierten Zelluloseethers enthält, indem der Zelluloseether dispergiert wird und der pharmazeutische wirksame Bestandteil dispergiert oder gelöst wird, beide in Wasser bei einer Temperatur über der vorbestimmten Temperatur;
(C) Beschichten der Kornkerne mit der Beschichtungsflüssigkeit, um beschichtete Körner zu erhalten; und
(D) Trocknen der beschichteten Körner.

2. Verfahren nach Anspruch 1,
bei dem das Pulver des Zelluloseethers eine derartige Korngrößenverteilung hat, daß zumindest 90 Gew.-% der Partikel durch ein Sieb mit einer Maschenweite von 149 µm passiert.

3. Verfahren nach Anspruch 1,
bei dem die Beschichtungsflüssigkeit 0,1 bis 50 Gew.-% des pharmazeutisch wirksamen Bestandteils enthält, bezogen auf das Gewicht des Zelluloseethers.

## Revendications

1. Procédé de préparation d'un médicament sous forme solide granulaire à libération progressive dont chaque granule est composée de :
(a) une granule-noyau formée d'un support comportant au moins une proportion importante d'un excipient ; et
(b) une couche de revêtement à la surface des granules, cette couche étant formée d'un éther de cellulose insoluble dans l'eau au-dessus d'une température prédéterminée, ladite couche de revêtement contenant en outre un ingrédient pharmaceutiquement actif,
ce procédé comportant les étapes suivantes :
(A) granulage d'un support composé d'au moins une proportion importante dudit excipient pour former lesdites granules-noyau;
(B) préparation d'un liquide de revêtement renfermant en dispersion de 5 à 30 pour cent en poids de l'éther de cellulose dispersé, par dispersion dudit éther de cellulose et par dispersion ou dissolution dudit ingrédient pharmaceutiquement actif, les deux dans de l'eau à une température supérieure à ladite température prédéterminée ;
(C) revêtement des granules-noyau par le liquide de revêtement pour former les granules revêtues ; et
(D) séchage des granules revêtues.

2. Procédé selon la revendication 1, selon lequel la poudre d'éther de cellulose présente une distribution de dimensions particulaires telle qu'au moins 90 pour cent en poids des particules passe au travers d'un tamis ayant des ouvertures de malle de 149 µm.

3. Procédé selon la revendication 1, dans lequel le liquide de revêtement renferme de 0,1 pour cent à 50 pour cent en poids de l'ingrédient pharmaceutiquement actif, basé sur le poids de l'éther de cellulose
